# EUROPEAN PATENT SPECIFICATION

(11) **EP 2 968 922 B1**
(45) Date of publication and mention of the grant of the patent: **15.08.2018**
(21) Application number: 14763554.4
(22) Date of filing: 14.03.2014
(51) Int. Cl.: A61N 1/05, A61N 5/06, A61B 5/042, A61B 5/04, A61B 5/026, A61B 5/06

(54) **COMBINATION PROBE FOR LOCALIZING, RECORDING AND STIMULATING A TARGET TISSUE**
KOMBINATIONSSONDE ZUR LOKALISIERUNG, AUFZEICHNUNG UND STIMULATION EINES ZIELGEWEBES
SONDE MIXTE PERMETTANT DE LOCALISER, D'ENREGISTRER ET DE STIMULER UN TISSU CIBLE

(30) Priority: 15.03.2013 US 201361788204 P; 02.04.2013 SE 1350409
(43) Date of publication of application: 20.01.2016
(73) Proprietor: Wårdell, Karin, 582 18 Linköping (SE)
(72) Inventor: Wårdell, Karin, 582 18 Linköping (SE)
(74) Representative: Nobeli Business Support AB
(86) International application number: PCT/SE2014/050314
(87) International publication number: WO 2014/142741

(56) References cited:
- EP-A1- 1 062 973
- KR-A- 20120 088 501
- US-A1- 2002 045 832
- US-A1- 2002 045 832
- US-A1- 2007 156 126
- US-A1- 2013 030 274
- US-B1- 6 304 784
- US-B1- 6 718 196
- US-B1- 7 883 536
- GUK BAE KIM ET AL.: 'Cortical Mapping of the Optically Evoked Responses in Channelrhodopsin-2 Mouse Model' 33RD ANNUAL INTERNATIONAL CONFERENCE OF THE IEEE EMBS 30 August 2011 - 03 September 2011, BOSTON, MASSACHUSETTS USA, pages 6769 - 6772, XP032320236

## Description

### Technical field

The present invention relates generally to a combination probe for localizing, recording and operatively stimulating a target tissue. More particularly, the technical field relates to neurosurgery.

### Background of invention

Deep brain stimulation (DBS) involves neurosurgery to implant electrodes directly into specific brain regions. A probe is used for preparing a path into the target tissue, so that the DBS-electrode may be deployed to the intended site. A battery pack is surgically deployed under the skin, usually in the chest, and wires link the battery pack to the DBS-electrodes, which deliver a continuous current of electricity to alter abnormal patterns of nerve activity.

The vast majority of centers use electrophysiological mapping techniques to finalize target selection during the implantation of deep brain stimulationlead for the treatment of Parkinson's disease and tremor. Deep brain stimulation is today used for alleviation of movement disorders (e.g Parkinson's disease, essential tremor, dystonia), but brain stimulation (does not have to be deep) is currently increased toward other disorders such as to prevent, alleviate or treat for example pain, and psychiatric illness.

Mapping strategies vary greatly across centers, but may be broadly categorized into those that use microelectrode or semi micro electrode techniques to optimize position prior to implantation and macro stimulation through a macro electrode or the DBS lead, and those that rely solely on macro stimulation and its threshold for clinical effects (benefits and side effects).

Today, there are two commercial methods available for intra operative recording; micro electrode recording (MER) and impedance measurements.

Micro electrode recording is the most common intra operative measurement method during deep brain stimulation. In order to minimize targeting error, studies with the recommendation of using microelectrode recording in place of intra operative imaging to arrive at the target location have been published. Implanting centers commonly use intra operative microelectrode recording to provide additional information and to better ensure placement at the desired target.

Micro electrodes or semi micro electrodes are used to characterize precisely the target nucleus and its boundaries prior to deployment of a macro electrode/DBS electrode. The physiological characteristics of the target subthalamic nucleus (STN), globus pallidus internus (GPi), ventral intermedius nucleus of the thalamus (Vim), cingulate cortex for depression and other, including the firing rate and pattern of its neurons, and those of surrounding structures, allow its positive identification. Moreover, the nature of the evoked responses of its neurons, e.g. movement-evoked responses, or local field potential (LFP), further allows characterization of the topography within the nucleus.

Impedance determines the voltage response of electrodes to current flow during recording and determines the current flow in response to voltage during stimulation.

Haemorrhagic complications carry by far the highest risk of devastating neurological outcome in functional neurosurgery. Well established patient factors include increased age and a history of hypertension. Surgical methodologies associated with haemorrhage include the use of MER and incursion by the trajectory into a sulcus or ventricle bleeding is common (variation of 0-30.4%) when MER is used during DBS implantation (Zrinzo *et al.,* 2012). The sharp tip profile associated with MER may be one explanation to the haemorrhage complications.

Research oriented techniques for intra operative navigation are optical methods, and there are two slightly different methods patented by Giller (US 6,567,690) and Wardell (EP 1146827), respectively.

US 6,567,690 describes a method and apparatus for probe localization in brain matter. The method comprises determining a deployment point for an object at a desired localisation within a tissue. The measuring use reflectance properties and near infrared light (NIRS).

EP1146827 uses reflectance spectroscopy and describes an apparatus for destroying a predetermined defined volume in a tissue, such as generating a lesion in for example the brain. The apparatus uses Laser Doppler perfusion monitoring (LDPM) for recording the tissue blood flow and tissue type.

According to WHO, neurological and psychiatric illness will increase rapidly in the next few decades, and many of the signs and symptoms seen in patients today arise as a consequence of disordered activity in neural circuits.

In view of the above, deep brain stimulation is an important technology for treating neurological, pain, and psychiatric illness. Since one of the most common complications is haemorrhage there is a need of a probe that minimizes the bleeding risk. Moreover, improved navigation of the probe is also important for securing successful deployment of electrodes into the target area.

### Summary of the invention

The present invention describes a novel combination probe for preparing a path into a target tissue, so that a DBS-electrode may be deployed into the intended target site.

The present probe minimizes the risk for haemorrhagic complications, i.e., reduces the bleeding risk during the deployment and navigation into a target tissue. This object is achieved with a probe according to the independent claim 1.

In one embodiment the present probe comprises a modified MER electrode in combination with at least one pair of optical fibres, coupled to a light source and a recording unit, respectively, thus providing a more accurate and secure probe for neurosurgery. The light source may be any light source for example a laser.

The present combination probe is not limited to a specific system; it may be used in any suitable commercial analysis, monitoring and/or navigation system.

An object of the present invention is to provide an improved and more accurate probe for use in neurosurgery, for example in deep brain stimulation. The probe is not limited to be used in deep brain areas, other areas also count.

This object is achieved by means of a combination probe according to the appended claims.

In one embodiment should the present combination probe be used for preparing a path into a target tissue, so that a DBS-electrode may be deployed into the intended target site. The probe comprises an essentially tube shaped outer housing accommodating a centred tube shaped inner portion for receiving an elongated conducting element serving as an electrode. At least one optical fibre pair is arranged in close proximity to the tube shaped inner portion, wherein one of the fibre pair are operatively connected to for example a laser source. The other fibre of the fibre pair is connected to a detector unit, for example for intra cerebral guidance during functional neurosurgery.

According to the invention, the elongated conducting element serving as an electrode is a MER electrode.

In one embodiment the fibers are aligned straight along the tube. Thereby the construction may be very much simplified. It may also be important to control the distance between the fibres, e.g. so that the fibres of each pair are closely arranged along each other.

The present invention provides a probe, comprising a MER electrode and optical fibres, utilizing optical sources such as Laser Doppler (LD) and/or diffuse reflectance spectroscopy (DRS).

In one embodiment is the combination probe used for localizing (by optics), recording and operatively stimulating tissue, by a MER electrode in the target, i.e., the electrophysiology is recorded. In another embodiment it is also possible to stimulate the tissue by using a MER electrode and to see how the tissue reacts or responds to the stimulation by optics and clinical evaluation.

In one embodiment Laser doppler may be used and the source of light in this case may be in the range of 700-900 nm, for example 780 nm. In the embodiment where reflectance spectroscopy is used the wavelength may be in the range of 400-1000 nm.

In one embodiment the navigation is monitored.

According to the invention, the elongated conducting element serving as an electrode is a MER electrode, and the tip of the elongated conducting element serving as an electrode is adjustable in the axial direction of the probe.

In one embodiment the optical probe may also be used separately before insertion of a MER electrode in order to record the blood flow and reduce the risk for haemorrhage in the measurement site or along the trajectory.

In one embodiment is a micro gun (Elekta MicroDrive, St. Jude) or a micro drive or similar supporting equipment used together with the optical probe (i.e., the present probe without a MER-electrode) to enable implantation of an electrode in the brain tissue. The measurement may be performed at positions from the cortex to the target region.

The tip is in one embodiment rounded for not damaging the tissue during deployment.

In yet another embodiment the probe is connected to a controllable energy source, for example a battery

In yet an embodiment, biocompatible glue is attached to the proximal end of the probe and stabilizes and fixes the optical fibers in the right position.

In one embodiment the tip diameter is more than 0.9 mm for not generating bleedings. The tip diameter is defined as the entire probe tip diameter.

In one embodiment is the combination probe connected to an energy source.

Another object is to provide a method for localizing, recording and operatively stimulating a target tissue, said method comprising the steps of:
- drilling a hole at a predetermined site in a skull
- moving the probe to the pre drilled hole via a navigation system, mechanical or by a hand driven mechanical device,
- deploying the probe into the pre drilled hole, with or without a supporting device,
- holding the probe in a certain position, measuring and recording the micro vascular perfusion and the total backscattered light intensity (TLI) at predetermined points from the target,
- push the electrode forward and record if the microcirculation is within a valid level,
- repeating the previous step on the next level if necessary for targeting the target tissue,
- stimulating the target tissue, and
- removing the probe.

While recording if the microcirculation is within a valid level the electrode is held still at the position of measurement.

In one embodiment is the navigation to the target tissue monitored. The monitoring equipment is any commercial equipment suitable for this purpose.

In one embodiment markers for different cells, organelles or blood may be extracted from the spectra, for example markers for Hb, HbO₂ or SO₂, chromophores, e.g. lipofysin, melanin, neuromelanin, cytochromes. Other suitable markers may also be used.

In one embodiment is the target tissue brain tissue.

In one embodiment the method described herein is for preventing, alleviating and/or treat neuro physiological or neuro associated disorders.

Common neurological and psychiatric conditions are for example Parkinson's disease (PD), essentiell tremor, dystonia, obsessive compulsive disorder, Gilles de la Tourettes syndrome, Alzheimers and depression. But brain stimulation (does not have to be in deep areas) is currently increased toward other disorders such as to prevent, alleviate or treat for example pain, and psychiatric illness.

Yet an object of the present invention is to provide a system comprising the combination probe described in the attached claims and above. Any commercial system that may be used in combination with the present probe is comprised in the present invention.

The system for localizing and operatively stimulating a target tissue may comprise:
- a combination probe as disclosed herein,
- a navigation system or a hand driven mechanical device for moving the combination probe via a pre drilled hole in a skull,
- a means for measuring and recording the micro vascular perfusion and the total backscattered light intensity (TLI) at predetermined points in the target,
- a means for pushing the electrode forward and recording if the microcirculation is within a valid level,
- a means for stimulating the target tissue.

In one embodiment the system is connected to an analysis system.

Yet another object of the present invention is use of the probes described above for localizing and operatively stimulating a target tissue.

In one embodiment is the probes used for a target tissue that is brain tissue.

In yet another embodiment is the probes described above used for alleviating, preventing and/or treat neurophysiological or neuro associated disorders.

The present technique/method may avoid bleedings by combining MER with laser Doppler (LD) measurements in one probe. At the same time LD may act as an intraoperative navigation tool as grey- and white matter may be distinguished with high resolution. DRS (TLI) also provides the opportunity to perform extraction of chromophore blood markers from the spectra, such as haemoglobine (Hb), deoxy heamoglobine (HbO₂), SO₂, Lipofuscin, melanin, neuromelanin, cytochromes etc.

Further aspects and embodiments are defined in the appended claims, which are specifically incorporated herein by reference.

### Brief description of drawings

The invention is now described, by way of example, with reference to the accompanying drawings, in which:
Figure 1 shows a schematic view of a combination probe according to the present invention.
Figure 2a shows a cross sectional view of the probe and figure 2b shows an axial sectional view of the probe 1.

### Detailed description

In the following, the present invention will be described in more detail by way of embodiments and with reference to the accompanying drawings.

Other features and uses of the invention and their associated advantages will be evident to a person skilled in the art upon reading the description and the examples.

It is to be understood that this invention is not limited to the particular embodiments shown here. The following examples are provided for illustrative purposes and are not intended to limit the scope of the invention since the scope of the present invention is limited only by the appended claims and equivalents thereof.

If nothing else is defined, any terms and scientific terminology used herein are intended to have the meanings commonly understood by those of skill in the art to which this invention pertains.

The term "about" as used in connection with a numerical value throughout the description and the claims denotes an interval of accuracy, familiar and acceptable to a person skilled in the art. Said interval is ± 10 %.

Standard protocol for neurostimulation with DBS involves a multipolar electrode placed within a target area of the brain, commonly the STN. The individual DBS electrode contacts are mounted within a non-rigid insulating carrier and delivered with a rigid stylet through an insertion tube, using stereotactic technique. Once the electrode is in the appropriate location, the insertion tube is removed, the electrode is fixated at the burr hole, and the rigid internal stylet is removed.

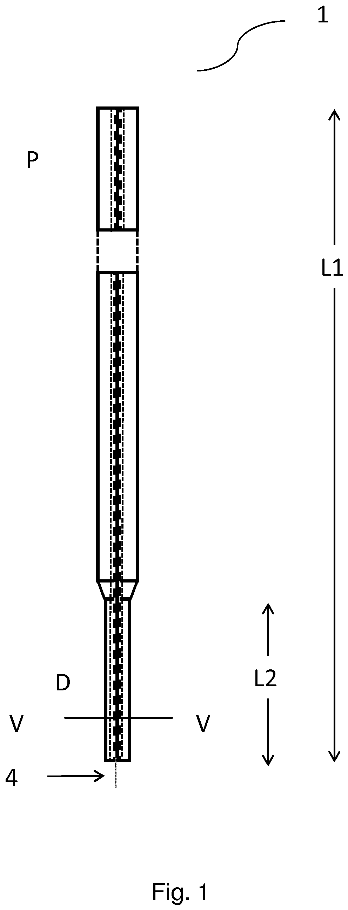

Figure 1 shows a schematic drawing of the outer dimensions of the combination probe 1 of the present invention. The probe 1 is suitable to common commercial navigation systems used today, for example the Leksell Stereotactic System (LSS), Brown Robert Wells (BRW) or commercial frameless systems. The probe 1 is essentially tube shaped, having a distal D and a proximal 2 end. The essentially tube shaped probe 1 tapers in the proximal end 2. The proximal end 2 comprises a tip 4, which is the end of the probe that enters the brain tissue. The total length L1 of the probe is in this embodiment about 190 mm long, and the tapered portion L2 is about 20-40 mm long. The diameter of the wider portion of the probe is in this embodiment about 2.1-2.2 mm and the diameter of the tapered portion is in this embodiment about 1.5-1.6 mm. The probe is connected to an energy source via proximal end of the probe.

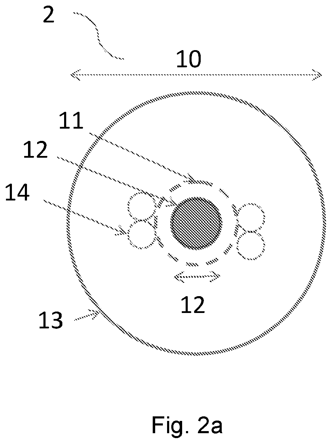

Figure 2a shows a cross sectional (V-V in Fig. 1) view of the proximal end portion 2 of the probe 1. The total diameter 10 of the outer tube shaped housing 13 is in this embodiment about 1.5 mm. Thereby the tip diameter, defined as the entire probe tip diameter, is more than 0.9 mm for not generating bleedings. The probe 1 also comprises a centred tube shaped inner portion 11 having a diameter in this embodiment about 0.7-0.9 mm. The tube shaped inner portion 11 receives and surrounds a MER electrode 12 having in this embodiment a diameter of about 0.66 mm, other diameters are also valid. Optical fibres 14 are arranged in close proximity to the tube shaped inner portion 11.

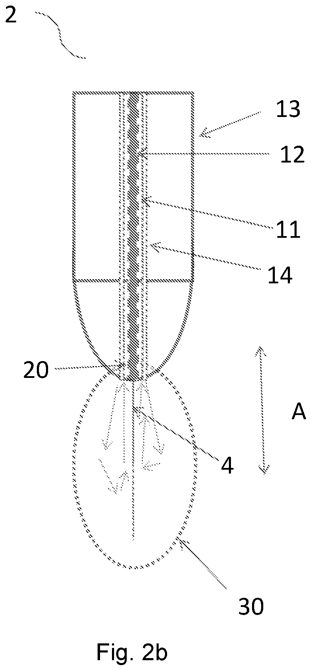

Figure 2b shows an axial sectional view of a portion of the proximal end portion 2 of the probe 1. The outer tube shaped housing 13 accommodates the fibres 14, and a tube shaped inner portion 11 comprises the MER-electrode 12 as shown in the figure. The MER tip 4 is adjustable in 1-3 mm steps in the axial direction A. However, other embodiments of the probe may have a MER-tip that is adjustable in 0.1-1 mm steps. The MER-electrode is measuring characteristics of the surroundings for identifying and localizing the target. The MER-electrode must be absolutely still in its position during each measurement step, otherwise there is a risk for miscalculations. The tube shaped inner portion 11 is surrounded by at least one pair of optical fibres 14 (∅ = e.g. 125µm or 140µm or 250µm) going in parallel to the tube all the way to the bottom of the probe (meaning a forward looking probe). Each pair of fibres is placed next to each other or on each side of the tube shaped inner portion 11 or in a circle around. The fibres of the present invention may be arranged in any way and not limited to the arrangement described here. One fibre from each pair is connected to a laser source and the other to a detector unit. The laser source may be a low power laser, but other laser sources are also possible to use. No separate tubes are necessary for the optical fibres 14. 30 shows the optical sampling volume. The numerical aperture of the optical fibres may be e.g. 0.22 or 0.37.

Biocompatible glue (20) is attached and polished into and onto the rounded end portion of the proximal end 2. The glue is fixating and stabilizing the positioning of the fibres. In this embodiment the rounded tip is having a diameter over 0.9 mm, but may be less than that in other embodiments. The fact that the tip is rounded makes it less sharp and easier to navigate through the tissue compared to other tips and minimizes therefore the risk of puncturing a vessel.

In order to perform LD measurements of the micro vascular blood flow the probe needs to be fixed at one position. A hand driven mechanical device (instead of an electric one) that may both change the position of the entire probe 1, and also the position of the MER tip 4. During the measurement the probe and its electrode is fixed at the site.

The probe and laser Doppler system (e.g. a Periflux, Perimed AB) are calibrated in order to show blood flow values in the range between 0-999. After calibration the system presents 250 units in Motility standard on the visualisation unit. Normal values in white matter are < 50 units, in cortex the values may range but are normally < 200 units, but may go up to 999 units when crossing a sulci and in the vicinity to a small vessel. Values in the deep brain structures STN, Vim and GPi are normally < 100. Other structures in the brain may be between other intervals.

### Surgery procedure

During surgery, the probe is moved to a site in the brain by a hand driven mechanical device fixed to the LSS. Once in place the blood flow is measured by sending out laser light in one fibre and detecting the backscattered light in the neighbouring fibre(s). The detected signal is processed to a blood flow value = <v>*CMBC (concentration of moving blood cells). In addition the total backscattered light intensity (TLI in the range of 0-10.0 units) of the laser Doppler system may be used to determine the tissue type (grey matter, white matter, and blood vessel). These values are stored together with the blood flow values.

If the blood flow value is above a predetermined threshold (e.g. 200 units) or TLI-signal is extremely low a warning signal is given (sound or blinking light in presentation panel) before deployment of the MER tip 4. If no warning is given, the MER tip 4 may be pushed forward in 1mm steps (1, 2, 3 mm) and the recordings be done in a safe way. The blood flow is measured during this procedure, and if the blood flow value changes more than a predefined value a new warning is given in order to indicate a potential bleeding or a vessel in the vicinity of the tip. In that case the MER tip could be withdrawn with the manual mechanical device. Measurement results - raw data or summarized ones will be presented online alone the trajectory on the patient's anatomical images (for example MRI) in order to assist the neurologists/neurosurgeons in deciding about the final implantation site.

The optical probe may be pushed down in order to occlude the bleeding, but might also be fixed at the spot (point) to occlude the bleeding. What the surgeon does depends on the situation.

Visualisation of blood flow, TLI, chromophores, SO₂ and MER signals are stored in relation to anatomy.

The MR images are presented together with a modelled probe and the current measurement site. The TLI, chromophores, SO₂, MER signal and blood flow signals are presented together or one at the time in the same panel. Presentations may be done in axial, coronal, sagital and "the probes view".

The present invention discloses a novel combination probe that counteracts some of the serious drawbacks, such as haemorrhage, of prior art. The probe is compatible with commercial navigation and monitoring systems used today. The present probe detects the surrounding tissue type more accurate due to its inventive combination of optical sources together with a MER electrode, thereby providing a superior probe.

## Claims

1. A combination probe (1) for localizing, recording and operatively stimulating a target tissue, said probe (1) comprising an essentially tube shaped outer housing (13) accommodating a centred tube shaped inner portion (11) for receiving an elongated conducting element serving as an electrode (12), wherein the tip (4) of the elongated conducting element serving as an electrode is adjustable in the axial direction (A) of the probe, and wherein at least one optical fibre pair (14) is arranged in close proximity to the tube shaped inner portion (11) towards the distal end (D), wherein one fibre of the fibre pair is operatively connected to
- a light source, and the other to a
- a detector unit, for intra-cerebral guidance during functional neurosurgery, wherein the light source is laser Doppler and/or diffuse reflectance spectroscopy (DRS), and wherein the elongated conducting element serving as an electrode is a MER electrode.

2. A combination probe according to claim 1, wherein the tip diameter is more than 0.9 mm.

3. A combination probe according to any of claim 1-2, wherein the probe is connected to a controllable energy source.

4. A system for localizing and operatively stimulating a target tissue, said system comprising:
- a combination probe (1) according to any one of claims 1-3
- a navigation system or a hand driven mechanical device for moving the combination probe via a pre drilled hole in a skull,
- a means for measuring and recording the micro vascular perfusion and the total backscattered light intensity (TLI) at predetermined points in the target,
- a means for pushing the electrode forward and recording if the microcirculation is within a valid level,
- a means for stimulating the target tissue.

5. The system according to claim 4 further comprising an analysis system.

6. The system according to any of claims 4-5 wherein DRS also provides the opportunity to perform extraction of chromophore blood markers from the spectra, for example markers for Hb, HbO₂ or SO₂.

## Patentansprüche

1. Kombinationssonde (1) zur Lokalisierung, Aufzeichnung und operativen Stimulation eines Zielgewebes, wobei die Sonde (1) ein im Wesentlichen rohrförmiges äußeres Gehäuse (13) umfasst, das einen zentrierten rohrförmigen inneren Anteil (11) beherbergt, um ein längliches leitendes Element aufzunehmen, das als Elektrode (12) dient, wobei die Spitze (4) des länglichen leitenden Elements, das als Elektrode dient, in der axialen Richtung (A) der Sonde anpassbar ist, und wobei mindestens ein optisches Faserpaar (14) in enger Nähe zu dem rohrförmigen inneren Anteil (11) in Richtung des distalen Endes (D) angeordnet ist, wobei eine Faser des Faserpaares funktional mit
- einer Lichtquelle und die andere mit
- einer Detektoreinheit verbunden ist, für intrazerebrale Führung während funktionaler Neurochirurgie,
wobei die Lichtquelle Laser-Doppler- und/oder diffuse Reflektanzspektroskopie (DRS) ist, und wobei das längliche leitende Element, welches als Elektrode dient, eine MER-Elektrode ist.

2. Kombinationssonde nach Anspruch 1, wobei der Spitzendurchmesser mehr als 0,9 mm beträgt.

3. Kombinationssonde nach einem der Ansprüche 1 bis 2, wobei die Sonde mit einer steuerbaren Energiequelle verbunden ist.

4. System zum Lokalisieren und funktionalen Stimulieren eines Zielgewebes, wobei das System umfasst:
- eine Kombinationssonde (1) nach einem der Ansprüche 1 bis 3;
- ein Navigationssystem oder eine handbetriebene mechanische Vorrichtung zum Bewegen der Kombinationssonde mittels eines vorgebohrten Lochs in einem Schädel,
- ein Mittel zum Messen und Aufzeichnen der mikrovaskulären Perfusion und der gesamten rückgestreuten Lichtintensität (TLI) an vorbestimmten Punkten in dem Ziel,
- ein Mittel zum Vorwärtsschieben der Elektrode und Aufzeichnen, falls die Mikrozirkulation innerhalb einer gültigen Höhe ist,
- ein Mittel zum Stimulieren des Zielgewebes.

5. System nach Anspruch 4, ferner umfassend ein Analysesystem.

6. System nach einem der Ansprüche 4 bis 5, wobei DRS auch die Gelegenheit zur Durchführung von Extraktion von chromophoren Blutmarkern aus den Spektren bietet, beispielsweise Markern für Hb, HbO₂ oder SO₂.

## Revendications

1. Sonde mixte (1) permettant de localiser, d'enregistrer et de stimuler fonctionnellement un tissu cible, ladite sonde (1) comprenant un logement externe sensiblement en forme de tube (13) accueillant une partie interne en forme de tube (11) centrée permettant d'accueillir un élément conducteur allongé faisant office d'électrode (12), la pointe (4) de l'élément conducteur allongé faisant office d'électrode étant ajustable dans la direction axiale (A) de la sonde, et au moins une paire de fibres optiques (14) étant agencée au voisinage immédiat de la partie interne en forme de tube (11) vers l'extrémité distale (D), une fibre de la paire de fibres étant reliée fonctionnellement à
- une source de lumière, et l'autre à
- une unité de détection, pour un guidage intracérébral pendant une opération de neurochirurgie fonctionnelle
la source de lumière étant un Doppler laser et/ou une spectroscopie de réflectance diffuse (DRS), et l'élément conducteur allongé faisant office d'électrode étant une électrode MER.

2. Sonde mixte selon la revendication 1, dans laquelle le diamètre de pointe est supérieur à 0,9 mm.

3. Sonde mixte selon l'une quelconque des revendications 1 et 2, dans laquelle la sonde est connectée à une source d'énergie réglable.

4. Système permettant de localiser et de stimuler fonctionnellement un tissu cible, ledit système comprenant :
- une sonde mixte (1) selon l'une quelconque des revendications 1 à 3
- un système de navigation ou un dispositif mécanique entraîné manuellement permettant de déplacer la sonde mixte par l'intermédiaire d'un trou préalablement percé dans un crâne,
- un moyen permettant de mesurer et d'enregistrer la perfusion microvasculaire et l'intensité lumineuse rétrodiffusée (TLI) totale en des points prédéfinis dans la cible,
- un moyen permettant de pousser l'électrode vers l'avant et d'enregistrer si la microcirculation est dans un niveau valide,
- un moyen pour stimuler le tissu cible.

5. Système selon la revendication 4 comprenant en outre un système d'analyse.

6. Système selon l'une quelconque des revendications 4 et 5, dans lequel la DRS offre également la possibilité d'extraire des marqueurs sanguins de chromophores présents dans les spectres, par exemple des marqueurs pour Hb, HbO₂ ou SO₂.
